# EUROPEAN PATENT SPECIFICATION

(11) **EP 0 526 503 B1**
(45) Date of publication and mention of the grant of the patent: **04.06.1997**
(21) Application number: 91908011.9
(22) Date of filing: 09.04.1991
(51) Int. Cl.: A61K 49/00, G01N 24/00, G01N 31/00, A61B 5/05, A61K 33/40

(54) **POLYMERS AS CONTRAST MEDIA FOR MAGNETIC RESONANCE IMAGING**
POLYMERE UND IHRE VERWENDUNG ALS KONTRASTMITTEL BEI DER BILDERZEUGUNG MIT MAGNETISCHER RESONANZ
POLYMERES UTILISES COMME MILIEUX DE CONTRASTE EN IMAGERIE PAR RESONANCE MAGNETIQUE

(30) Priority: 10.04.1990 US 507125
(43) Date of publication of application: 10.02.1993
(60) Divisional application: 95111117.8
(73) Proprietor: IMARX PHARMACEUTICAL CORP., Tucson, AZ 85719 (US)
(72) Inventor: Unger, Evan C., Tucson, Arizona 85749 (US)
(74) Representative: Stebbing, Peter John Hunter
(86) International application number: US9102429
(87) International publication number: WO9115753

(56) References cited:
- EP-A- 0 118 281
- EP-A- 0 184 899
- EP-A- 0 228 692
- EP-A- 0 284 549
- EP-A- 0 409 351
- WO-A-85/04330
- WO-A-89/11874
- WO-A-90/01953
- US-A- 4 069 306
- US-A- 4 719 098
- US-A- 4 729 892
- US-A- 4 775 522
- US-A- 4 822 594
- US-A- 4 838 274
- US-A- 4 849 210
- US-A- 4 871 716
- US-A- 4 925 648
- US-A- 4 933 441
- US-A- 4 996 041

## Description

This invention relates to the field of magnetic resonance imaging, more specifically to the use of polymers in combination with contrast agents for magnetic resonance imaging.

There are a variety of imaging techniques that have been used to diagnose disease in humans. One of the first imaging techniques employed was X-rays. In X-rays, the images produced of the patients' body reflect the different densities of body structures. To improve the diagnostic utility of this imaging technique, contrast agents are employed to increase the density between various structures, such as between the gastrointestinal tract and its surrounding tissues. Barium and iodinated contrast media, for example, are used extensively for X-ray gastrointestinal studies to visualize the esophagus, stomach, intestines and rectum. Likewise, these contrast agents are used for X-ray computed tomographic studies to improve visualization of the gastrointestinal tract and to provide, for example, a contrast between the tract and the structures adjacent to it, such as the vessels or lymph nodes. Such gastrointestinal contrast agents permit one to increase the density inside the esophagus, stomach, intestines and rectum, and allow differentiation of the gastrointestinal system from surrounding structures.

Magnetic resonance imaging (MRI) is a relatively new imaging technique which, unlike X-rays, does not utilize ionizing radiation. Like computed tomography, MRI can make cross- sectional images of the body, however MRI has the additional advantage of being able to make images in any scan plane (i.e., axial, coronal, sagittal or orthogonal). Unfortunately, the full utility of MRI as a diagnostic modality for the body, particularly in the abdominal and pelvic region, is hampered by the lack of an effective gastrointestinal contrast agent. Without such an agent, it is often difficult using MRI to differentiate the intestines from, for example, adjacent soft tissues and lymph nodes. If better contrast agents were available, the overall usefulness of MRI as an imaging agent would improve, and the diagnostic accuracy of this modality in the gastrointestinal region would be greatly enhanced.

MRI employs a magnetic field, radiofrequency energy and magnetic field gradients to make images of the body. The contrast or signal intensity differences between tissues mainly reflect the T1 and T2 relaxation values and the proton density (effectively, the free water content) of the tissues. In changing the signal intensity in a region of a patient by the use of a contrast medium, several possible approaches are available. For example, a contrast medium could be designed to change either the T1, the T2 or the proton density.

A paramagnetic contrast agent such as Gd-DTPA causes longitudinal relaxation to shorten T1. This increases the signal intensity on T1-weighted images. A superparamagnetic contrast agent such as ferrites works predominantly on transverse relaxation causing a shortening of T2 and decreasing signal intensity on T2-weighted images. A contrast agent could also work by altering the proton density, specifically by decreasing the amount of free water available that gives rise to the signal intensity.

Agents that increase the signal intensity from the lumen compared to the native contents are termed positive contrast agents. A number of these have been examined as contrast agents for MRI. These include fats and oils (Newhouse et al., Radiology, 142(P):246 (1982)), which increase signal as a result of their short T1, long T2 and high intrinsic proton density, as well as various paramagnetic agents that increase signal by decreasing the T1 of water protons. Examples of such paramagnetic agents include Gd-DTPA (Kornmesser et al., Magn. Reson. Imaging, 6:124 (1988), and Laniado et al., AJR, 150:817 (1988)), Gd-DOTA (Hahn et al. Magn. Reson. Imaging, 6:78 (1988)), Gd-oxalate (Runge, V.M. et al., Radiology, 147:789 (1983)), Cr-EDTA (Runge, V.M. et al., Physiol. Chem. Phys. Med. NMR, 16:113 (1984)), Cr-Tris-acetylacetonate (Clanton et al., Radiology, 149:238 (1983)), ferric chloride (Young et al., CT, 5:543 (1981)), ferrous gluconate (Clanton et al., Radiology, 153:159 (1984)), ferric ammonium citrate and ferrous sulfate (Wesbey et al., Radiology, 149:175 (1983) and Tscholakoff et al., AJR, 148:703 (1987)) as well as iron complexes (Wesbey et al., Magn. Reson. Imaging, 3:57 (1985) and Williams et al., Radiology, 161:315 (1986)).

Alternatively, agents that decrease the signal intensity from the lumen are termed negative contrast agents. Examples include particulate iron oxides (Hahn et al., Radiology, 164:37 (1987), Widder et al., AJR, 149:839 (1987)) which decrease signal via T2 shortening, as well as gas-evolving materials (Weinreb et al., J. Comput. Assist. Tomogr., 8:835 (1984)) and perfluorocarbons (Mattrey et al., AJR, 148:1259 (1987)) which act through changes in the proton density. It should be recognized that all paramagnetic substances at sufficiently high concentrations can also result in a decrease in signal intensity via T2 shortening.

The existing MRI contrast agents all suffer from a number of limitations when employed as oral gastrointestinal agents. Positive contrast agents increase the image noise arising from intrinsic peristaltic motions and motions imposed via respiration or cardiovascular action. Positive contrast agents such as Gd-DTPA are subject to the further complication that the signal intensity depends upon the concentration of the agent as well as the pulse sequence used. Absorption of contrast agent from the gastrointestinal tract complicates interpretation of the images, particularly in the distal portion of the small intestine, unless sufficiently high concentrations of the paramagnetic species are used (Kornmesser et al., Magn. Reson. Imaging, 6:124 (1988)). Negative contrast agents by comparison are less sensitive to variation in pulse sequence and provide more consistent contrast. However at high concentrations, particulates such as ferrites can cause magnetic susceptibility artifacts which are particularly evident in the colon where the absorption of intestinal fluid occurs and the superparamagnetic material may be concentrated. Negative contrast agents typically exhibit superior contrast to fat, however on T1-weighted images, positive contrast agents exhibit superior contrast versus normal tissue. Since most pathological tissues exhibit longer T1 and T2 than normal tissue, they will appear dark on T1-weighted and bright on T2-weighted images. This would indicate that an ideal contrast agent should appear bright on T1-weighted images and dark on T2-weighted images. None of the currently available MRI contrast media for use with the gastrointestinal tract meet these dual criteria.

Toxicity is another problem with the existing contrast agents. With any drug there is some toxicity, the toxicity generally being dose related. With the ferrites there are often symptoms of nausea after oral administration, as well as flatulence and a transient rise in serum iron. The paramagnetic contrast agent Gd-DTPA is an organometallic complex of gadolinium coupled with the complexing agent diethylene triamine pentaacetic acid. Without coupling, the free gadolinium ion is highly toxic. The peculiarities of the gastrointestinal tract, wherein the stomach secretes acids and the intestines release alkalines, raise the possibility of decoupling and separation of the free gadolinium from the complex as a result of these changes in pH during gastrointestinal use. Certainly, minimizing the dose of either gastrointestinal contrast agent, whether paramagnetic or superparamagnetic, is important for minimizing any potential toxic effects.

New and/or better contrast agents useful in magnetic resonance imaging, particularly in the imaging of the gastrointestinal tract but also in the imaging of other regions of the body such as through the vasculature, are needed. The present invention is directed to this important end.

The present invention is directed to a contrast medium for magnetic resonance imaging, said contrast medium comprising an aqueous solution or suspension of a biocompatible polymer in admixture with a MRI contrast agent. The contrast agent may further comprise as contrast agent, paramagnetic, superparamagnetic and/or proton density contrast agents. The polymer and contrast agent admixtures may also comprise, if desired, biocompatible gases, preferably gases such as air, oxygen, carbon dioxide, nitrogen, xenon, neon and/or argon.

The subject invention may be used to provide an image of an internal region of a patient, especially an image of the gastrointestinal region of the patient to whom a contrast medium as above has been administered, which method comprises scanning the patient using magnetic resonance imaging to obtain visible images of the region.

The aspects of the invention will become more apparent from the following detailed description when taken in conjunction with the following drawings.

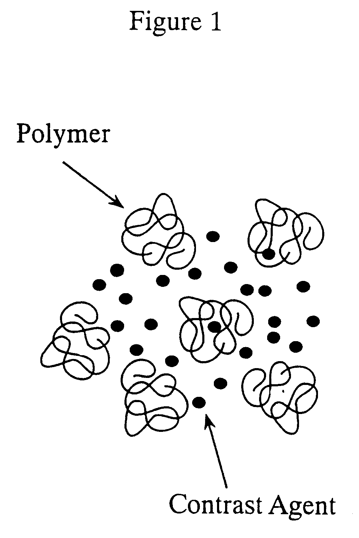

Fig. 1 is a diagrammatic view of a contrast medium in accordance with the present invention.

Preferably, the polymer chosen is one which has a relatively high water binding capacity. Also preferably, the polymer has limited ability for ion complexation. Where imaging of the gastrointestinal region is desired, preferably the polymer chosen is one which is not substantially absorbed from or degraded within the gastrointestinal region.

The exclusion and concentration effect is illustrated diagrammatically in Figure 1, with the polymer being represented by the squiggly lines and the contrast agent being represented by the solid dots.

Exemplary polymers for use in the present invention include the polymer hyaluronic acid as well as polymers of glucuronic acid, galacturonic acid and mannuronic acid.

For reasons of diagnostic efficacy, other preferable polymers include polygalacturonic acid.

The polymers of the present invention are employed in an aqueous solution in admixture with conventional contrast agents. By admixture, it is meant that the contrast agent is simply added to the polymer-containing medium, and is not chemically bound to the polymer by a covalent linkage. Electrostatic interactions or hydrogen bonding may, however, exist between the polymer and contrast agents, and such associations are considered to be within the ambit of the term admixture.

Numerous contrast agents are well known to those skilled in the art and include, for example, paramagnetic, superparamagnetic and proton density contrast agents.

Exemplary paramagnetic contrast agents suitable for use in the subject invention include stable free radicals (such as, for example, stable nitroxides), as well as compounds comprising transition, lanthanide and actinide elements covalently or noncovalently bound to complexing agents or to proteinaceous macromolecules. Preferable elements include Gd(III), Mn(II), Cu(II), Cr(III), Fe(II), Fe(III), Co(II), Er(II), Ni(II), Eu(III) and Dy(III). More preferably, the elements include Gd(III), Mn(II), Cu(II), Fe(II), Fe(III), Eu(III) and Dy(III), especially Gd(III). Preferable complexing agents include, for example, diethylenetriamine-pentaacetic acid (DTPA), ethylene-diaminetetraacetic acid (EDTA), 1,4,7,10-tetraazacyclododecane-N,N',N',N'''-tetraacetic acid (DOTA), 1,4,7,10-tetraazacyclododecane-N,N',N''-triacetic acid (DO3A), 3,6,9-triaza-12-oxa-3,6,9-tricarboxymethylene-10-carboxy-13-phenyl-tridecanoic acid (B-19036), hydroxybenzylethylene-diamine diacetic acid (HBED), N,N'-bis(pyridoxyl-5-phosphate)ethylene diamine, N,N'-diacetate (DPDP), 1,4,7-triazacyclononane-N,N',N''-triacetic acid (NOTA), 1,4,8,11-tetraazacyclotetradecane-N,N'N'',N'''-tetraacetic acid (TETA) and kryptands (that is, macrocyclic complexes). More preferably, the complexing agents are DTPA, DOTA, DO3A and kryptands, most preferably DTPA. Preferable proteinaceous macromolecules include albumin, collagen, polyarginine, polylysine, polyhistidine, γ-globulin and β-globulin. More preferably, the proteinaceous macromolecules comprise albumin, polyarginine, polylysine, and polyhistidine. Most preferably, the paramagnetic contrast agents employed in the present invention are Gd(III)-DTPA, Gd(III)-DOTA, Gd(III)-DO3A, or Gd(III)-kryptands, especially Gd(III)-DTPA.

Exemplary superparamagnetic contrast agents suitable for use in the subject invention include ferro- or ferrimagnetic compounds, such as pure iron, magnetic iron oxide (such as magnetite), γ-Fe₂O₃, manganese ferrite, cobalt ferrite and nickel ferrite.

Exemplary proton density contrast agents include perfluorocarbons.

The polymer and contrast agent admixtures of the subject invention may also be employed, if desired, in admixture with biocompatible gases. In the case of both negative and positive contrast agents, such gases often serve to increase the efficacy of the contrast medium. The gas can be bubbled through the medium using conventional techniques. Any biocompatible gas is suitable for use in the present invention. Numerous such gases are well known to those skilled in the art. Exemplary biocompatible gases include, air, oxygen, carbon dioxide, nitrogen, xenon, neon and argon.

Wide variations in the amounts of the polymer, and the contrast agent and/or gas, can be employed in the contrast medium of the invention. Preferably, however, the polymer is present in a concentration of at least about 1%, by weight, more preferably, between about 5% and about 50%, by weight, and generally most preferably at about 40%, by weight. Of course, as those skilled in the art would recognize, within these parameters the optimum polymer concentration will be influenced by the molecular weight of the polymer, its water binding capacity, as well as other characteristics of the particular polymer employed. Also, preferably, in the case of paramagnetic and proton density contrast agents, the contrast agent is present in a concentration of at least about 0.1 millimolar, more preferably between about 0.5 and about 2 millimolar, most preferably at about 1 millimolar. In the case of superparamagnetic agents, the concentration is preferably at least about 1 micromolar, more preferably between about 5 and about 50 micromolar, and most preferably is about 10 micromolar. Where gas is employed, preferably at least about 20 psi is bubbled through the solution for about one minute prior to administration, more preferably between about 30 or about 50 psi, most preferably about 40 psi.

The present invention is useful in imaging a patient generally, and/or in specifically diagnosing the presence of diseased tissue in a patient. The imaging process of the present invention may be carried out by administering a contrast medium of the invention to a patient, and then scanning the patient using magnetic resonance imaging to obtain visible images of an internal region of a patient and/or of any diseased tissue in that region. By region of a patient, it is meant the whole patient or a particular area or portion of the patient. The contrast medium is particularly useful in providing images of the gastrointestinal region, but can also be employed more broadly such as in imaging the vasculature or in other ways as will be readily apparent to those skilled in the art. The phrase gastrointestinal region or gastrointestinal tract, as used herein, includes the region of a patient defined by the esophagus, stomach, small and large intestines and rectum. The phrase vasculature, as used herein, denotes the blood vessels in the body or in an organ or part of the body. The patient can be any type of mammal, but most preferably is a human.

As one skilled in the art would recognize, administration may be carried out in various fashions, such as intravascularly, orally, rectally, etc., using a variety of dosage forms. When the region to be scanned is the gastrointestinal region, administration of the contrast medium of the invention is preferably carried out orally or rectally. The useful dosage to be administered and the particular mode of administration will vary depending upon the age, weight and the particular mammal and region thereof to be scanned, and the particular medium of the invention to be employed. Typically, dosage is initiated at lower levels and increased until the desired contrast enhancement is achieved. Various combinations of biocompatible polymers may be used to modify the relaxation behavior of the medium or to alter properties such as the viscosity, osmolarity or palatability (in the case of orally administered materials). In carrying out the method of the present invention, the contrast medium can be used alone, or in combination with other diagnostic, therapeutic or other agents. Such other agents include excipients such as flavoring or coloring materials. In addition, if desired, the contrast media of the invention may be encapsulated in liposomes or in other delivery vehicles. The polymer or polymer and contrast agent and/or gas admixture may be sterilized by autoclaving prior to use, if desired.

The magnetic resonance imaging techniques which are employed are conventional and are described, for example, in D.M. Kean and M.A. Smith, Magnetic Resonance Imaging: Principles and Applications, (William and Wilkins, Baltimore 1986). Contemplated MRI techniques include, but are not limited to, nuclear magnetic resonance (NMR) and electronic spin resonance (ESR). The preferred imaging modality is NMR.

As will be apparent to those skilled in the art, the polymer admixture with contrast agent and/or gas, when employed in magnetic resonance imaging, may operate as a T1, T2 or proton density contrast medium, depending upon the type of polymer used, the molecular weight of the polymer, the concentration of the polymer, the type of contrast agent mixed with the polymer, the type of MRI modality chosen, and the details of the pulse sequence employed for MRI imaging, and all such mechanisms of operation are considered to be within the ambit of the present invention.

The media of the present invention have been shown to be extremely useful as contrast enhancement media in magnetic resonance imaging, particularly in imaging of the gastrointestinal region. By employing the polymers alone or by combining a contrast agent with the polymers in accordance with the present invention, lower overall concentrations of the contrast agents may be used to achieve the same, or in many cases a better degree of, contrast enhancement results. This has benefits not only in terms of toxicity, by avoiding the use of large amounts of the potentially toxic contrast agents, but also in terms of cost, since less of the often more expensive conventional contrast agents are used. Furthermore, in the case of negative contrast agents based on superparamagnetic particles, magnetic susceptibility artifacts will be reduced through the ability to use a lower dose of the contrast agent. These and other advantages described herein of the present invention will be readily apparent to those skilled in the art, upon reading the present disclosure.

The present invention is further described in the following Examples. These Examples are not to be construed with as limiting the scope of the appended Claims.

### EXAMPLES:-

### Example 1

A 2% aqueous solution of the polymer poly-galacturonic acid in admixture with Mn (II) was prepared. The relaxivity (1/T1 and 1/T2) of the solution was then tested in vitro using a Toshiba MRT-50A 0.5 Tesla (T) whole body scanner. The 1/T1 was measured at 41.0 ± 1.92 mmol⁻¹sec⁻¹, and the 1/T2 was measured at 79.41 ± 3.20 mmol⁻¹sec⁻¹.

### Example 2

A 0.5% aqueous solution of the polymer poly-galacturonic acid was prepared. To the solution was then added 0.1nM Mn (II). The relaxivity (1/T1 and 1/T2) of the solution was tested in vitro using a Toshiba MRT-50A 0.5 Tesla (T) whole body scanner. The 1/T1 was measured at 1.20 ± 0.056 sec⁻¹, and the 1/T2 was measured at 4.78 ± 0.001 sec⁻¹.

### Example 3

The procedure of Example 2 were substantially followed, except that sodium hydroxide was added to the solution in an amount sufficient to raise the pH from 2 (as in Example 2), to a pH of 4, 6, 7 and 8, respectively. The relaxivity (1/T1 and 1/T2) of the solutions at these various pH levels was tested in vitro using a Toshiba MRT-50A 0.5 Tesla (T) whole body scanner. The results are shown below in Table 1.

**Table 1**

| Relaxivities at 0.5T For Water/Polygalacturonic Acid/Mn (II) Mixtures At Varying pH Levels | | |
|---|---|---|
| Sample pH | 1/T1(1/sec) | 1/T2(1/sec) |
| pH 4 | 4.15 ± 0.208 | 6.78 ± 0.143 |
| pH 6 | 4.10 ± 0.192 | 7.941 ± 0.320 |
| pH 7 | 3.73 ± 0.215 | 6.29 ± 0.240 |
| pH 8 | 4.11 ± 0.546 | 6.64 ± 0.268 |

Various modifications of the invention in addition to those shown and described herein will be apparent to those skilled in the art from the foregoing description. Such modifications are also intended to fall within the scope of the appended Claims.

## Claims

1. A contrast medium for magnetic resonance imaging comprising an aqueous solution or suspension of a biocompatible polymer selected from polygalacturonic acid, polyglucuronic acid, polymannuronic acid and hyaluronic acid in admixture with an MRI contrast agent.

2. A contrast medium of Claim 1 wherein the polymer is polygalacturonic acid.

3. A contrast medium of Claim 1 or Claim 2 wherein the MRI contrast agent is selected from paramagnetic, superparamagnetic and proton density contrast agents.

4. A contrast medium of Claim 3 wherein the contrast agent is a paramagnetic agent.

5. A contrast medium of Claim 4 wherein the paramagnetic agent is Mn(II).

6. A contrast medium for use in a method of providing an image of an internal region of a patient to whom a contrast medium of of any preceding has been administered, which method comprises scanning the patient using magnetic resonance imaging to obtain visible image of the said region.

## Patentansprüche

1. Kontrastmittel zur magnetischen Resonanzabbildung, das eine wäßrige Lösung oder Suspension eines biokompatiblen Polymers, ausgewählt aus Polygalacturonsäure, Polyglucoronsäure, Polymannuronsäure und Hyaluronsäure in einer Mischung mit einem MRI-Kontrastmittel, umfaßt.

2. Kontrastmittel nach Anspruch 1, bei dem das Polymer Polygalacturonsäure ist.

3. Kontrastmittel nach Anspruch 1 oder Anspruch 2, bei dem das MRI-Kontrastmittel aus paramagnetischen, superparamagnetischen und protonenempfindlichen Kontrastmitteln ausgewählt wird.

4. Kontrastmittel nach Anspruch 3 bei dem das Kontrastmittel ein paramagnettsches Mittel ist.

5. Kontrasmittel nach Anspruch 4, bei dem das paramagnetische Mittel Mn(II) ist.

6. Kontrastmittel zur Anwendung bei einem Verfahren zur Verfügungstellung eines Bildes eines internen Bereichs eines Patienten, dem ein Kontrastmittel nach irgendeinem der vorherigen Ansprüche verabreicht wurde, wobei das Verfahren das Scannen des Patienten umfaßt, wobei magnetische Resonanzabbildung angewendet wird, um ein sichtbares Bild diese Bereichs zu erhalten.

## Revendications

1. Milieu de contraste pour imagerie par résonance magnétique, comprenant une solution ou suspension aqueuse d'un polymère bio-compatible choisi parmi l'acide polygalacturonique, l'acide polyglucuronique, l'acide polymannuronique et l'acide hyaluronique en mélange avec un agent de contraste d'imagerie par résonance magnétique (MRI).

2. Milieu de contraste selon la revendication 1, dans lequel le polymère est l'acide polygalacturonique.

3. Milieu de contraste selon la revendication 1 ou la revendication 2, dans lequel l'agent de contraste MRI est choisi parmi les agents de contraste paramagnétiques, supra paramagnétiques et modifiant la densité protonique.

4. Milieu de contraste selon la revendication 3, dans lequel l'agent de contraste est un agent paramagnétique.

5. Milieu de contraste selon la revendication 4, dans lequel l'agent paramagnétique est Mn (II).

6. Milieu de contraste destiné à être utilisé dans un procédé pour fournir une image d'une région interne d'un patient auquel un milieu de contraste de l'une quelconque des revendications précédentes a été administré, lequel procédé comprend la scanographie du patient à l'aide de l'imagerie par résonance magnétique pour obtenir une image visible de ladite région.
